## Europäisches Patentamt

## European Patent Office

## Office européen des brevets

(11) Publication number: **0 354 200**
**A2**

(12) **EUROPEAN PATENT APPLICATION**

(21) Application number: **89870114.9**

(22) Date of filing: **24.07.89**

(51) Int. Cl.⁵: **G 01 N 33/569**

(30) Priority: **25.07.88 US 223483**

(43) Date of publication of application:
**07.02.90 Bulletin 90/06**

(84) Designated Contracting States:
**AT BE CH DE ES FR GB GR IT LI LU NL SE**

(71) Applicant: **SMITHKLINE BIOLOGICALS S.A.**
**Rue du Tilleul, 13**
**B-1320 Genval Rixensart (BE)**

(72) Inventor: **Thiriart, Clotilde**
**Drève de la Ferme, 34**
**B-1970 Wezembeek-Oppem (BE)**

**Bruck, Claudine**
**Avenue du Gris Moulin, 53**
**B-1310 La Hulpe (BE)**

(74) Representative: **Tasset, Gérard**
**Smithkline Biologicals s.a. Rue de l'Institut 89**
**B-1330 Rixensart (BE)**

(54) **HIV diagnostic method and reagent.**

(57) HIV infection is diagnosed by assaying for presence of anti-CD4 antibodies in a bodily fluid of a person.

Bundesdruckerei Berlin

**Description**

## HIV DIAGNOSTIC METHOD AND REAGENT

### Field of the Invention

This invention relates to HIV and to methods of diagnosis and treatment thereof.

### Background of the Invention

The Human Immunodeficiency Virus (HIV) is the etiologic agent of AIDS, AIDS Related Complex (ARC) and associated immune disorders. To date, at least three strains or types of HIV have been identified, HIV-1, HIV-2 and HIV-3.

The T cell glycoprotein, CD4, has been shown to be the receptor for binding and infection of T4+ lymphocytes by HIV.

Maddon et al., PCT patent application WO8801304, disclose the structure of the CD4 glycoprotein and a cDNA coding there for.

Ozturk et al., J. Clin Immunol. 7:130 (1987), Dorsett et al., Ann J. Med. 78:621 (1985), and Stricker et al., Nature 327:710 (1987), report presence of anti-lymphocyte antibodies in HIV-1 infected patients.

It is an object of the present invention to provide a method for diagnosing HIV infection, which method can be used to supplement existing HIV diagnostic methods.

### Summary of the Invention

This invention, in one aspect, is a method for diagnosing HIV infection in a person which comprises contacting a sample of a bodily fluid of the person with a CD4 glycoprotein or an immunologically equivalent derivative thereof and assaying for binding of anti-CD4 antibodies in the sample to the CD4 glycoprotein or immunologically equivalent derivative thereof.

In a related aspect, this invention is a diagnostic device comprising a solid support having a CD4 glycoprotein or an immunologically equivalent derivative thereof bound thereto.

In further related aspect, this invention is a kit comprising such device and, in separate containers, a labelled anti-CD4 antibody or a labelled anti-human immunoglobulin antibody and a substrate for detecting the label.

### Detailed Description of the Invention

For use in the invention, a full length CD4 glycoprotein or an immunologically equivalent derivative thereof is employed. Immunologically equivalent derivatives thereof are proteins or oligo- or polypeptides which are cross-reactive with anti-CD4 antibodies produced in a human infected with HIV.

The preferred protein for use in the invention is sCD4, which is a derivative of CD4 lacking the transmembrane and cytoplasmic domains. Such protein is disclosed in Maddon et al., PCT W08801304. A sequence for sCD4 is as follows.

```
                10                      30                      50
CAAGCCCAGAGCCCTGCCATTTCTGTGGGCTCAGGTCCCTACTGCTCAGCCCCTTCCTCC


                70                      90                     110
CTCGGCAAGGCCACAATGAACCGGGGAGTCCCTTTTAGGCACTTGCTTCTGGTGCTGCAA
              MetAsnArgGlyValProPheArgHisLeuLeuLeuValLeuGln


               130                     150                     170
CTGGCGCTCCTCCCAGCAGCCACTCAGGGAAAGAAAGTGGTGCtGGGCAAAAAAGGGGAT
LeuAlaLeuLeuProAlaAlaThrGlnGlyLysLysValValLeuGlyLysLysGlyAsp
     -9                          -1 +1


               190                     210                     230
ACAGTGGAACTGACCTGTACAGCTTCCCAGAAGAAGAGCATACAATTCCACTGGAAAAAC
ThrValGluLeuThrCysThrAlaSerGlnLysLysSerIleGlnPheHisTrpLysAsn


               250                     270                     290
TCCAACCAGATAAAGATTCTGGGAAATCAGGGCTCCTTCTTAACTAAAGGTCCATCCAAG
SerAsnGlnIleLysIleLeuGlyAsnGlnGlySerPheLeuThrLysGlyProSerLys


               310                     330                     350
CTGAATGATCGCGCTGACTCAAGAAGAAGCCTTTGGGACCAAGGAAACTTCCCCCTGATC
LeuAsnAspArgAlaAspSerArgArgSerLeuTrpAspGlnGlyAsnPheProLeuIle


               370                     390,                    410
ATCAAGAATCTTAAGATAGAAGACTCAGATACTTACATCTGTGAAGTGGAGGACCAGAAG
IleLysAsnLeuLysIleGluAspSerAspThrTyrIleCysGluValGluAspGlnLys


               430                     450                     470
GAGGAGGTGCAATTGCTAGTGTTCGGATTGACTGCCAACTCTGACACCCACCTGCTTCAG
GluGluValGlnLeuLeuValPheGlyLeuThrAlaAsnSerAspThrHisLeuLeuGln
                                                  104


               490                     510                     530
GGGCAGAGCCTGACCCTGACCTTGGAGAGCCCCCCTGGTAGTAGCCCCTCAGTGCAATGT
GlyGlnSerLeuThrLeuThrLeuGluSerProProGlySerSerProSerValGlnCys
```

```
            550                  570                  590
AGGAGTCCAAGGGGTAAAAACATACAGGGGGGGAAGACCCTCTCCGTGTCTCAGCTGGAG
ArgSerProArgGlyLysAsnIleGlnGlyGlyLysThrLeuSerValSerGlnLeuGlu


            610                  630                  650
CTCCAGGATAGTGGCACCTGGACATGCACTGTCTTGCAGAACCAGAAGAAGGTGGAGTTC
LeuGlnAspSerGlyThrTrpThrCysThrValLeuGlnAsnGlnLysLysValGluPhe
151

            670                  690                  710
AAAaTAGACATCGTGGTGCTAGCTTTCCAGAAGGCCTCCAGCATAGTCTATAAGAAAGAG
LysIleAspIleValValLeuAlaPheGlnLysAlaSerSerIleValTyrLysLysGlu
                                          183


            730                  750                  770
GGCGAACAGGTGGAGTTCTCCTTCCCACTCGCCTTTACAGTTGAAAAGCTGACGGGCAGT
GlyGluGlnValGluPheSerPheProLeuAlaPheThrValGluLysLeuThrGlySer


            790                  810                  830
GGCGAGCTGTGGTGGCAGGCGGAGAGGGCTTCCTCCTCCAAGTCTTGGATCACCTTTGAC
GlyGluLeuTrpTrpGlnAlaGluArgAlaSerSerSerLysSerTrpIleThrPheAsp


            850                  870                  890
CTGAAGAACAAGGAAGTGTCTGTAAAACGGGTTACCCAGGACCCTAAGCTCCAGATGGGC
LeuLysAsnLysGluValSerValLysArgValThrGlnAspProLysLeuGlnMetGly


            910                  930                  950
AAGAAGCTCCCGCTCCACCTCACCCTGCCCCAGGCCTTGCCTCAGTATGCTGGCTCTGGA
LysLysLeuProLeuHisLeuThrLeuProGlnAlaLeuProGlnTyrAlaGlySerGly


            970                  990                  1010
AACCTCACCCTGGCCCTTGAAGCGAAAACAGGAAAGTTGCATCAGGAAGTGAaCCTGGTG
AsnLeuThrLeuAlaLeuGluAlaLysThrGlyLysLeuHisGlnGluValAsnLeuVal


            1030                 1050                 1070
GTGATGAGAGCCACTCAGCTCCAGAAAAATTTGACCTGTGAGGTGTGGGGACCCACCTCC
ValMetArgAlaThrGlnLeuGlnLysAsnLeuThrCysGluValTrpGlyProThrSer
```

4

```
      1090                    1110                    1130
CCTAAGCTGATGCTGAGCTTGAAACTGGAGAACAAGGAGGCCAAAGGTCTCGAAGCGCGAG
ProLysLeuMetLeuSerLeuLysLeuGluAsnLysGluAlaLysValSerLysArgGlu


      1150                    1170                    1190
AAGGCGGTGTGGGTGCTGAACCCTGAGGCGGGCATGTGGCAGTGTCTGCTGAgTGACTCG
LysAlaValTrpValLeuAsnProGluAlaGlyMetTrpGlnCysLeuLeuSerAspSer


      1210                    1230                    1250
GGACAGGTCCTGCTGGAATCCAACATCAAGGTTCTGCCCACATGGTCCACCCCGGtgtaa
GlyGlnValLeuLeuGluSerAsnIleLysValLeuProThrTrpSerThrProValEnd
351                                                       369


      1270
tggcgcctctaga
```

This sequence is substantially identical to the sequence disclosed by Maddon et al., except that in the Maddon et al. sequence, the glutamine herein designated (-)2 is therein designated (+)1 and the lysine herein designated (+)1 is therein designated asparagine, (+)3.

Other derivatives of CD4, e.g., a V1 containing protein or a protein containing a portion of V1, can also be used provided such cross reacts with anti-CD4 antisera as produced by HIV infected animals. Such cross reactivity is readily determined by comparing reactivity of sera from an HIV-infected animal, preferably a human, with sCD4 with reactivity of the same sera with the CD4 derivative. Such immunologically equivalent protein or derivative will comprise at least about 6 amino acids from sCD4 and, more prefereably, will comprise at least about 10 amino acids from sCD4.

The proteins used in the invention can be prepared synthetically or by recombinant DNA techniques. The preferred method for producing sCD4 is by expression in recombinant CHO cells, as described by Deen et al., Nature 331:87 (1988). incorporated herein by reference.

In carrying out the diagnostic method of the invention, standard diagnostic methods are employed. These include radioimmunoassay, western blotting assay and enzyme linked immunosorbent assay (ELISA). Suitable methods are disclosed, for example, by Gallo et al., U.S. Patent 4,520,113, Blackburn et al., PCT WO8707957, Montagnier et al., U.S. Patent 4,708,818, Essex et al., U.S. 4,725,669 Debouck et al., U.S. patent application Serial Number 7-056,553 and Saxinger et al. U.S. Patent 4,661,445. An ELISA assay is preferred for its convenience. An exemplary assay employing multiple reagents is currently in commercial use, under the Trademark, HIVAGEN, by SmithKline Beckman Clinical Laboratories, King of Prussia, Pennsylvania. See, "Reliable Confirmation of Antibodies to HIV-1 with an Enzyme-linded Immunoassay Using Recombinant Antigens Derived From the HIV-1 gag, pol and env genes," by Valerie L. Ng, et al., submitted to N. Eng. J. Med. in July 1988.

In a typical ELISA, antigen is bound, i.e., adsorbed, bound, entrapped or otherwise affixed to a solid support, e.g., a membrane, filter, bead, column or plate. The patient sample, e.g., lymphatic fluid, cerebrospinal fluid, saliva, tears or preferably, serum, is then added. An enzyme-labelled antibody to the adsorbed antigen, such as one raised in a laboratory animal or one or more monoclonal antibodies, is then added. Alternatively, an enzyme-labelled antibody to human antibodies can be employed prior to or following addition of the sample. Finally, an enzyme substrate is added. The wells are then screened for binding of anti-CD4 antibodies from the patient sample by screening for enzymatic activity. The amount of enzymatic activity, in the case of labelled anti-CD4 antibody, is inversely proportional to the amount of antibody specific to the antigen in the patient sample and, in the case of labelled anti-human antibody antibody, is directly proportional. The amount of enzymatic activity is generally measurable by use of a colorimetric substrate and can be measured relative to control wells. For example, the following procedure can be used:

1. a NUNC Immunoplate I (Cat. No. 4-39454) is coated with sCD4 by loading 50µL of sCD4, 1 ug/ml ir phosphate buffered saline (pH 7) (PBS) and incubating overnight at 4 C.

2. the wells are incubated for 1 hour at 37 C with a saturation buffer (1% bovine serum albumim (BSA), 4% NSA and 0.1% Tween 20 in PBS);

3. serial dilutions of patient sera (1/500 to 1/5000) in saturation buffer are applied to the wells (50 µl/well) and incubated for 1 1/2 hour at 37 C;

4. biotinylated species specific anti-human immunoglobulins (whole antibody) (Amersham, Cat. No. RPN 1003) diluted 1/500 in saturation buffer are applied (50 µl/well) and incubated for 1 hour at 37 C.

5. streptavidin biotinylated horseradish peroxidase complex (Amersham, Cat. No. 1051) diluted 1/1000 in saturation buffer is applied (50 µl/well) and incubated at 37 C;

6. substrate OPDA (Sigma Chemical Co., St. Louis, Missouri, Cat. No. P8747) is added and incubated at 37 C.

Thus, this invention also comprises a diagnostic device comprising a solid support to which CD4 or an immunologically equivalent derivative thereof is attached. The solid support is preferably a plastic plate having wells in which the CD4 or derivative is adsorbed. This invention also comprises a kit comprising such device and, in separate containers, a labelled anti-CD4 antibody or a labelled anti-human immunoglobulin antibody and a substrate for detecting the label.

It has been found, as discussed below, that anti-sCD4 antibodies can appear early in the infection cycle and, in some cases, can be the first detectable indication of HIV infection. Moreover, the level of sCD4 antibodies generally increases with time following HIV infection. Therefore, the method of this invention is valuable as an early indicator of HIV infection and as a relative measure of the length of time since infection. Such information is valuable to physicians prescribing a medical course of action and to persons in high risk groups as an early indicator of HIV infection.

Preferably, the method of the invention is carried out in conjunction with other methods of diagnosing HIV infection. Particularly, it is preferred that the protein or peptide used in the method of the invention be used as part of a panel of reagents, as in the case of the HIVAGEN diagnostic test.

In the following study, in which data proving the utility of anti-sCD4 antibody assays as a diagnostic method of HIV infection was generated, an ELISA assay substantially as described above was employed. The antigen was sCD4 prepared in CHO cells as described by Deen et al., Nature 331:82 (1988). Briefly, the transmembrane domain of the cDNA reported by Maddon et al., cited above, was deleted by restricting the cDNA with Hpall at base pair 1252 and ligating thereto a linker which restored base pairs 1253 and 1257 and which placed a translation stop codon, TAA, after the C-terminal valine. This coding sequence was operatively linked to a SV40 early promoter and to a bovine growth hormone (BGH) polyadenylation region and co transfected with a gene for dihydrofolate reductase (DHFR) into a DHFR-deficient chinese hamster ovary (CHO) cell line.

Recombinant soluble (sCD4) was used to analyse the anti-CD4 antibodies occuring in sera of 253 HIV-1 seropositive individuals. These samples originated from a well documented group of Dutch homosexual HIV-1 seropositive patents (De Wolf, F. et al. Br. Med. J. 295:569 572; De Wolf, F. et al. J. Inf. Dis. in press (1988)) are were collected at different stages of the disease. All the samples from the Dutch group had been extensively characterized for the following parameters at the time of each sampling : anti-HIV-1 antibodies, p24 antigenemia to core HIV antigen, T4 cell count, T4/T8 ratio and clinical stage (CDC: Centers for Disease Control. MMWR 35:334-339 (1986)). Serial samples collected at various intervals (a few weeks to 4 months) were available from each patient. Sera from 120 seronegative persons, as well as from 9 HIV-1 infected chimpanzees (Gajdusek, D.C. et al. Lacet 1:55-56 (1985)), from 9 HIV-2 patients from various origins and from 10 healthy human volunteers immunized with recombinant vaccinia gp160 (Zagury, D. et al. Nature 332:728-731 (1988)) were also included in this study.

Soluble CD4 was directly adsorbed to the wells of microtiter plates and a 1/500 dilution of all the sera were tested for the binding to sCD4 in a EIA using biotin labelled anti-human immunoglobulins. The results summarized in Table I below, show that sera from 8-19% of the Dutch HIV-1 positive individuals contains anti-sCD4 activity. None of the other groups tested display this kind of reactivity. Interestingly, the reactivity to sCD4 is distributed unevenly among the seroconverted group (sera collected 11-12 months after HIV-1 seroconversion) (8%), the HIV-1 seropostive asymptomatic group (12.6%) and the seropositive ARC/AIDS group (19.4%).

Table I

Frequency of detectable anti-sCD4 antibodies in
human and chimps sera

| anti-sCD4 antibodies | | mean titer of anti-sCD4 antibodies |
| --- | --- | --- |
| Seroconverted (tested one year after seroconversion) | 5/60 (8 %) | 1/500 to 1/1000 |
| HIV-1 positive human (CDC II or III) | 20/157 (12,7 %) | 1/1000 to 1/5000 |
| HIV-1 positive humans (CDC IV) | 7/36 (19,4 %) | |
| HIV-2 positive human (clinical stage unknown) | 1/9 (11 %) | 1/5000 |
| HIV-1 infected Chimps | 0/10 | NA |
| Vaccinia env vaccinated Humans | 0/10 | NA |
| HIV-1 Seronegative Humans | 0/10 | NA |
| HIV-1 Seronegative Humans | 0/120 | NA |

The specificity of these antibodies to sCD4 as a possible minor contaminant of the sCD4 preparation was assessed by Western Blot analysis of several strongly sCD4 positive sera using purified sCD4 as antigen. These data (not shown) clearly indicated that sT4 is the major component recognized by human sera selected for the high reactive in the sCD4 EIA.

The anti-sCD4 reactivity of the positive human sera was contained at least in part, in the IgG fraction of the sera since purified IgGs from a HIV seropositive patient retained this reactivity (results not shown).

Preincubation of the coated sCD4 with high concentration of OK4A does not alter the anti-sCD4 reactivity of the tested human sera. This result rules out the possibility that the binding of human antibodies to sCD4 occurs through an indirect interaction involving CD4-gp120 anti-gp120 antibody complexes.

Moreover, preincubation of the wells coated with sCD4 with different dilutions of several highly positive human sera did not inhibit the subsequent fixation of monoclonal antibodies anti-Leu3a and OKT4A. These results show that the tested sera do not contain significant amounts of anti-sCD4 antibodies directed to the gp120 binding site of CD4. A possible antidiotypic origin of the observed anti-sCD4 reactivity is thus very unlikely.

No significant correlation between the presence of antibodies to sCD4, the clinical state and the CD4 cell count emerges from this comparison of the presence of antibodies to sCD4 in the Dutch homosexual group (1 specific sample from each patient) with the clinical and immunological status at the time of sampling. This suggests that anti-sCD4 antibodies 1) do not by themselves account for the depletion of CD4 cells 2) do not have an immediate adverse effect on the CD4 lymphocyte count. Moreover, antibodies to sCD4 are not sufficient, nor necessary to induce ARC/AIDS in HIV-1 seropositive patients. Nevertheless, in view of the association of increased anti-CD4 antibody titers with length of time post-infection and in view of the fact that the symptoms of AIDS and ARC are consistent with an immune response to T4+ lymphocytes, it is believed that anti-CD4 antibodies are involved in disease progression. Thus, removal of anti-CD4 antibodies, such as by extracorporeal passage of patient sera through a support having anti-CD4 antibodies bound thereto can be used in treatment of disease states associated with HIV infection.

Serial samples from 15 Dutch homosexual individuals including 5 individuals who seroconverted during the course of the follow-up are included in this study in order to analyze the temporal evolution of sCD4 antibodies and to try to correlate the appearance of sCD4 antibodies with other changing parameters. The obtained

EP 0 354 200 A2

results show that, in the seroconverted group (patients who seroconverted to HIV-1 during the course of the study), antibodies to sCD4 appeared at various times after seroconversion (Table II, below). Interestingly, one of the "seroconverted" patients even raised antibodies to sCD4 before mounting a detectable seroconversion to HIV-1 (Table II). The average titer of the anti-CD4 antibodies in the "seroconverted" group was lower (1/500 to 1/100) than in the "seropositive" group (1/1000 to 1/5000) (patients who were seropositive at the beginning of the study). This observation can be correlated with a lower percentage of sCD4 reactivity among "seroconverted" than among "seropositives" (see Table I) and raises the possibility that the anti-sCD4 antibody titer slowly increases over a time period of several years. Indeed, the moment of HIV-1 seroconversion of the "seropositive" group is unknown and could significantly exceed the delay (11-12 months) after HIV-1 seroconversion used for the screening of the "seroconverted" group. In most seroconverted patients, anti-cD4 antibody levels tended to increase with the greatest increase occuring during the first six months following seroconversion and with an irregular rise thereafter (14 sequential sera analyzed). Some of the patients showed an extremely irregular profile featuring peaks of anti-CD4 activity. In one patient, antibodies to sCD4 returned to an undetectable level 32 months after their first detection.

Table II

Antibody response to sCD4 in 5 out of 60 HIV-1 antibody seroconverted homosexual men

| Time before and after HIV-1 antibody seroconversion (months) | -6 | -3 | 0 | 3 | 6 | 0 | 12 |
|---|---|---|---|---|---|---|---|
| Number of anti-sCD4 positives (%) | 0/5 | 1/5 | 1/5 | 2/5 | 3/5 | 3/5 | 3/5 |

In summary, the obtained results show that a significant proportion of HIV-1 infected patients mount an immune response to soluble sCD4 antigen. Among the Dutch homosexual patient group analyzed in this study, 8 to 19% of the patients display anti-sCD4 reactivity, the frequency tending to be higher in the ARC/AIDS group. The observed titers range from 1/500 to 1/5000. Anti-CD4 antibodies were not detected in HIV negative human sera and in a limited series of sera from HIV-2 infected patients, from HIV-1 infected chimps and from vaccinia gp160 vaccinated individuals. Thus these data show that the method of the invention can be used to diagnose HIV-1 infection in humans.

The above description and Examples fully disclose the invention including preferred embodiments thereof. Modifications and improvements of the embodiments specifically disclosed herein are within the scope of the following claims.


**Claims**

1. A method for collecting data useful in diagnosing HIV infection in a person which comprises contacting a sample of a bodily fluid of the person with a CD4 glycoprotein or an immunologically equivalent derivative thereof.

2. The method of claim 1 wherein the CD4 protein or derivative is bound to a solid support.

3. The method of claim 2 which is an ELISA in which the binding of sample anti-CD4 antibody is directly assayed by treatment with labelled anti-human immunoglobulin antibody or is indirectly assayed by treatment with labelled anti-CD4 antibody.

4. The method of claim 3 in which the sample is a serum sample.

5. A diagnostic device comprising a solid support having CD4 or an immunologically equivalent derivative thereof bound thereto.

6. A kit comprising the device of claim 5 and, in separate containers, a labelled anti-CD4 antibody or a labelled anti-human immunoglobulin antibody and a substrate for detecting the label.